# EUROPÄISCHE PATENTSCHRIFT

(11) **EP 3 518 017 B1**
(45) Veröffentlichungstag und Bekanntmachung des Hinweises auf die Patenterteilung: **17.06.2020**
(21) Anmeldenummer: 18153149.2
(22) Anmeldetag: 24.01.2018
(51) Int. Cl.: G02B 23/24, A61B 1/00, G02B 23/26, G02B 6/00, A61B 1/07, A61B 5/00, F21V 8/00, G02B 6/06

(54) **VERFAHREN UND FASEROPTISCHES SYSTEM ZUR BELEUCHTUNG UND DETEKTION EINES OBJEKTS MIT LICHT**
METHOD AND OPTICAL FIBRE SYSTEM FOR ILLUMINATION AND DETECTION OF AN OBJECT WITH LIGHT
PROCÉDÉ ET SYSTÈME À FIBRE OPTIQUE DESTINÉS À ÉCLAIRER ET À DÉTECTER UN OBJET PAR LA LUMIÈRE

(43) Veröffentlichungstag der Anmeldung: 31.07.2019
(73) Patentinhaber: Technische Universität Dresden, 01069 Dresden (DE)
(72) Erfinder: CZARSKE, Jürgen, 01277 Dresden (DE); KUSCHMIERZ, Robert, 01099 Dresden (DE)
(74) Vertreter: Kailuweit & Uhlemann Patentanwälte Partnerschaft mbB

(56) Entgegenhaltungen:
- WO-A1-2010/004297
- WO-A1-2017/174596
- DONGGYU KIM ET AL: "Toward a miniature endomicroscope: pixelation-free and diffraction-limited imaging through a fiber bundle", OPTICS LETTERS, OPTICAL SOCIETY OF AMERICA, US, Bd. 39, Nr. 7, 1. April 2014 (2014-04-01), Seiten 1921-1924, XP001589259, ISSN: 0146-9592, DOI: 10.1364/OL.39.001921 [gefunden am 2014-03-24]
- MOONSEOK KIM ET AL: "Transmission matrix of a scattering medium and its applications in biophotonics", OPTICS EXPRESS, Bd. 23, Nr. 10, 18. Mai 2015 (2015-05-18), Seite 12648, XP055492687, ISSN: 2161-2072, DOI: 10.1364/OE.23.012648
- REZA NASIRI MAHALATI ET AL: "Resolution limits for imaging through multi-mode fiber", OPTICS EXPRESS, Bd. 21, Nr. 2, 28. Januar 2013 (2013-01-28), Seite 1656, XP055492717, ISSN: 2161-2072, DOI: 10.1364/OE.21.001656
- CONKEY DONALD B ET AL: "Lensless two-photon imaging through a multicore fiber with coherence-gated digital phase conjugation", INTERNATIONAL SOCIETY FOR OPTICAL ENGINEERING, SPIE, PO BOX 10 BELLINGHAM WA 98227-0010 USA, Bd. 21, Nr. 4, 30. April 2016 (2016-04-30) , Seite 45002, XP060071961, ISSN: 1083-3668, DOI: 10.1117/1.JBO.21.4.045002 [gefunden am 2016-04-18]

## Beschreibung

Die Erfindung betrifft ein Verfahren und ein faseroptisches System zur Beleuchtung und Detektion eines Objekts mit Licht, insbesondere für endoskopische und mikroskopische Anwendungen. Die Erfindung umfasst die in-situ Kalibrierung von faseroptischen Bildwellenleitern (Faserbündeln) und die Störungskorrektur von Beleuchtung oder Detektion.

Faseroptische Systeme in Form von flexiblen Endoskopen weisen eine hohe Zahl (bis zu mehreren 100000) einzelner, biegsamer optischer Fasern, z. B. Glasfasern, auf. Bei einer kohärenten Anordnung der Einzelfasern eines Faserbündels kann eine optische Bildübertragung vorgenommen werden. Vorteile gegenüber starren Endoskopen, bei denen die Bildinformation über ein Stablinsensystem weitergeleitet wird, liegen insbesondere in der Kompaktheit der haarfeinen Faserbündel und den flexiblen Einsatzmöglichkeiten, auch bei stark eingeschränkter Zugänglichkeit des Beobachtungsobjekts. Ein Nachteil flexibler Endoskope liegt darin, dass der erreichbare Bildkontrast relativ gering ist. Ursachen hierfür sind insbesondere die Pixelation des Faserbündels, das Übersprechen zwischen den Einzelfasern, das zu einer Reduktion der Bildschärfe führt (Blur-Effekt), und eine Multimodigkeit der Lichtwellenführung in den Einzelfasern (Speckle-Effekt). Die Verschlechterung der Bildqualität aufgrund der beschriebenen Störungen durch das Faserbündel kann mittels einer Modulationstransferfunktion beschrieben werden. Des Weiteren erlauben flexible Faserbündel nur die Übertragung von Intensitätsprofilen und deren Abbildung mittels Linsen (Nahfeldtechnik). Aufgrund der unterschiedlichen und unbekannten optischen Weglängen der Einzelfasern ist die Phaseninformation des übertragenen Lichts nicht direkt zugänglich, so dass das Fernfeld des übertragenen Lichts unbekannt und nicht gezielt einstellbar ist.

Eine Verkleinerung des Pixelabstands einer mit einem flexiblen Endoskop vorgenommenen Abbildung kann durch Integration einer Abbildungsoptik, in der Regel einer optischen Linse oder eines Linsensystems, erzielt werden, die die distale Ebene des Faserbündels zur Objektebene abbildet. Dadurch vergrößert sich allerdings die Baugröße des Endoskops erheblich, so dass die Mindestgröße für den optischen Zugang erhöht wird. Außerdem sind aufwändige Aufbau- und Verbindungstechniken zur Integration der Abbildungsoptik notwendig sowie zahlreiche Justageschritte. Ein weiterer Nachteil ergibt sich daraus, dass Faserbündel mit konventionellen Abbildungsoptiken nur zweidimensionale Messungen in der lateralen Ebene erlauben. Um eine Tiefeninformation zu erhalten, müssen z. B. Scanmethoden mit mechanisch verschiebbaren optischen Elementen oder elektrisch durchstimmbaren Optiken oder andere aufwändige Messverfahren (Triangulation mit zwei Faserbündeln) durchgeführt werden.

Zur Korrektur von Störungen, die durch Transmission durch optische Wellenleiter hervorgerufen werden, existieren verschiedene Ansätze. Die WO 2004/032386 A1 beschreibt ein Verfahren zur Korrektur polarisationsabhängiger Störungen in Lichtwellenleitern, wobei eine Kompensationsfunktion für die polarisationsabhängigen Störungen durch den Lichtwellenleiter berechnet wird, mittels derer zunächst ein verzerrtes elektrisches Eingangssignal berechnet wird, das in ein korrespondierend verzerrtes optisches Eingangssignal umgerechnet wird, welches zur Transmission durch den Lichtwellenleiter bestimmt ist. Zur Bestimmung der Kompensationsfunktion wird ein Detektor eingesetzt, der zur Messung des Signal-Rausch-Verhältnisses, der polarisationsabhängigen Dämpfung oder Modendispersion, der Bitfehlerhäufigkeit oder der Signaldispersion konfiguriert ist.

Aus dem Stand der Technik sind Methoden der adaptiven Optik bekannt, bei denen durch den Einsatz von Wellenfrontsensoren und -modulatoren Phasenaberrationen erfasst und ausgeglichen werden können. Beispielsweise in der DE 602 23 130 T2 sind derartige Verfahren zur Strahlsteuerung von Hochenergie-Laserstrahlen offenbart. In Cui, M. und Yang, C. Implementation of a digital optical phase conjugation system and its application to study the robustness of turbidity suppression by phase conjugation. Optics Express 18, Nr. 4 (2010), S. 3444 wird ein Verfahren der adaptiven Optik mit offenem Regelkreis vorgestellt, das als "digitale optische Phasenkonjugation" bezeichnet wird und zur Korrektur von Phasenverzerrungen durch ein optisch trübes, insbesondere biologisches Medium geeignet ist. Dabei werden eine CCD-Kamera als Sensor und ein Flächenlichtmodulator (spatial light modulator, SLM) als Aktor eingesetzt. Das beschriebene Verfahren erfordert es, dass jedes Pixel der Kamera ein virtuelles Bild auf einem korrespondierenden Pixel des SLM erzeugt, und umgekehrt. Das Verfahren verlangt dadurch eine aufwändige Kalibrierung und einen sehr hohen Justageaufwand. In Gu, R. Y., Mahalati, R. N. und Kahn, J. M. Design of flexible multimode fiber endoscope. Optics Express 23, Nr. 21 (2015), S. 26905 wird ein Endoskop mit biegsamen Multimodefasem beschrieben, dass unter Nutzung eines Teilreflektors am distalen Ende und eines SLM am proximalen Ende kalibriert werden kann. Die Kalibrierung des Endoskops und Messungen mit dem Endoskop können nicht gleichzeitig vorgenommen werden. Die Angaben "proximal" und "distal" charakterisieren Lagebeziehungen bezüglich des Faserbündels, wobei die proximale Seite des Faserbündels die der Beleuchtungsquelle zugewandte Stirnseite ist, und die distale Seite des Faserbündels die dem Objekt zugewandte Stirnseite ist.

In der US 8,585,587 B2 ist ein flexibles Endoskop beschrieben, bei dem mittels eines am proximalen Faserende angeordneten SLM die relative Phase des einfallenden Lichts verändert werden kann. Die durch die Fasern verursachte Phasenverschiebung bezüglich des einfallenden Lichts wird mittels eines Wellenfrontsensors oder Interferometers bestimmt. Dazu wird eine teilreflektive Beschichtung auf die distalen Faserenden aufgebracht. Die Bestimmung der Phasenverschiebung Δφ erfolgt dadurch nach zweimaligem Durchgang des Lichts durch das Faserbündel, gemessen wird also nicht die einfache Phasenverschiebung, sondern 2Δφ, jeweils modulo 2π. Die Messung der doppelten Phasenverschiebung hat den Nachteil, nicht eindeutig zu sein. Wird beispielsweise eine doppelte Phasenverschiebung um π gemessen, kann die einfache, durch das Faserbündel verursachte Phasenverschiebung π/2 oder - π/2 betragen. Die wahre einfache Phasenverschiebung kann nur in aufwändigen Verfahren ermittelt werden.

Um dies zu umgehen, wird in der US 2015/0015879 A1 vorgeschlagen, einen Multimode-Wellenleiter distal mittels eines virtuellen Lichtquellenbilds zu beleuchten. Betrachtet wird eine doppelt ummantelte Multimodefaser mit einem Monomodefaserkern, an deren distalem Ende ein holographisches Aufnahmematerial sowie ein punktreflexerzeugendes Objekt (wodurch eine virtuelle Lichtquelle hinter dem Objekt erzeugt wird) platziert ist. Bei Beleuchtung interferiert die durch die Monomodefaser transmittierte Welle mit dem von der virtuellen Lichtquelle ausgehenden Licht konstruktiv am Ort des Aufnahmematerials. Nachteilig ist die Position der virtuellen Lichtquelle festgelegt und kann nicht frei gewählt werden, und damit ist auch die Positionswahl für den realen Fokus stark eingeschränkt. Des Weiteren ist am distalen Faserende ein spezieller optischer Aufbau notwendig.

Aufgabe der vorliegenden Erfindung ist es daher, ein verbessertes Verfahren und ein zugehöriges faseroptisches System vorzuschlagen, mittels derer eine Korrektur der durch die Übertragung des von einer Beleuchtungsquelle emittierten Lichts durch ein Bündel optischer Fasern hervorgerufenen Störungen vorgenommen werden kann, ohne dass wiederholt aufwändige Justageschritte zur Kalibrierung des Systems durchgeführt werden müssen. Mit anderen Worten soll das Bündel optischer Fasern als kohärentes Phasenarray ("remote phased array") für Licht genutzt werden können.

Die Aufgabe wird gelöst durch ein Verfahren mit den Merkmalen des Anspruchs 1 und ein faseroptisches System mit den Merkmalen des Anspruchs 9. Weiterbildungen der Erfindung sind in Unteransprüchen angegeben.

Das erfindungsgemäße Verfahren zur Beleuchtung und Detektion eines Objekts mit Licht umfasst die Kalibrierung eines kohärenten Bündels optischer Fasern und die störungskorrigierte Beleuchtung oder störungskorrigierte Detektion des Objekts. Das kohärente Bündel optischer Fasern, beispielsweise Glasfasern, ist insbesondere biegsam gestaltet. Die Kalibrierung in Verfahrensschritt a) dient zumindest der Erfassung und Auswertung der Systemfunktion des Faserbündels, die durch die Transmissionsmatrix zwischen dem proximalen und dem distalen Ende des Faserbündels dargestellt werden kann und in der Literatur teilweise auch als "Übertragungsfunktion" bezeichnet wird. Im Verfahrensschritt b) wird die ermittelte Störung bei der Beobachtung des Objekts weitestgehend kompensiert. Die Störung kann beispielsweise als Verzerrung der Wellenfronten des Lichts bzw. als Deformation des Wellenfelds veranschaulicht werden. Die Beobachtung des Objekts umfasst dessen Beleuchtung und Detektion.

Mittels des erfindungsgemäßen Verfahrens können die Kalibrierung des Faserbündels und die störungskompensierte Beobachtung des Objekts vorteilhaft in-situ durchgeführt werden, so dass Änderungen der Systemfunktion, die beispielsweise durch Bewegung, Vibration oder Temperaturänderung hervorgerufen werden, ohne aufwändige manuelle Justage sogleich erfasst werden können.

Ein geordnetes Faserbündel wird als "kohärent" bezeichnet, wenn die Positionsbeziehung zwischen jeweils zwei Fasern des Bündels über die gesamte Länge des Bündels erhalten bleibt. Lagebeziehungen bezüglich des Faserbündels werden im Folgenden häufig durch die Angaben "proximal" und "distal" charakterisiert, wobei die proximale Seite des Faserbündels die dem Beleuchtungssystem zugewandte Stirnseite ist, und die distale Seite des Faserbündels die dem Objekt zugewandte Stirnseite ist.

Das erfindungsgemäße Verfahren umfasst folgende Unterverfahrensschritte zur Kalibrierung des Faserbündels in Verfahrensschritt a):
i) Proximale Beleuchtung mindestens einer frei wählbaren Einzelfaser des kohärenten Bündels optischer Fasern mit Licht;
ii) Zumindest teilweise Reflexion des Lichts an von den distalen Facetten der optischen Fasern des Bündels beabstandet angeordneten Mitteln zur teilweisen Reflexion des Lichts, so dass von dem reflektierten Licht ein mehr als eine Einzelfaser umfassender Bereich des Bündels optischer Fasern distal beleuchtet wird, wobei das reflektierte Licht dem von dem virtuellen Bild der beleuchteten Einzelfaser ausgehenden Licht entspricht;
iii) Detektion der räumlichen Intensitätsverteilung des reflektierten, durch die Übertragung mittels des distal beleuchteten Bereichs des Bündels optischer Fasern gestörten Lichts oder der räumlichen Intensitätsverteilung eines durch kohärente Überlagerung des reflektierten, durch die Übertragung mittels des distal beleuchteten Bereichs des Bündels optischer Fasern gestörten Lichts mit einer Referenzwelle erzeugten Interferenzmusters;
iv) Auswertung der detektierten Daten zumindest zur direkten Extraktion der einfachen Phasenverschiebung ΔΦ des reflektierten, durch die Übertragung mittels des distal beleuchteten Bereichs des Bündels optischer Fasern gestörten Lichts;
v) Ermittlung einer die Übertragungseigenschaften des distal beleuchteten Bereichs des Bündels optischer Fasern widerspiegelnden Systemfunktion;
vi) Wiederholung der Verfahrensschritte i) bis v) zur Ermittlung einer die Übertragungseigenschaften des gesamten Bündels optischer Fasern widerspiegelnden Systemfunktion.

Für die Kalibrierung des Faserbündels wird ausgenutzt, dass auf distaler Seite des Faserbündels bei Reflexion des durch eine einzelne Faser des Faserbündels geleiteten Lichts an den Mitteln zur teilweisen Reflexion auf der nicht dem Faserbündel zugewandten Seite der Mittel zur teilweisen Reflexion ein virtuelles Bild der Einzelfaser entsteht, wobei das reflektierte Licht als von dieser annähernd punktförmigen, virtuellen Quelle ausgehend aufgefasst werden kann. Es erfolgt also eine Beleuchtung distal von der virtuellen Lichtquelle aus, wobei die Anzahl der distal beleuchteten Fasern des Faserbündels vom Abstand zwischen der virtuellen Lichtquelle und den distalen Facetten des Faserbündels abhängig ist. Die Anwendung der annähernd punktförmigen, virtuellen Lichtquelle weist Analogien zum aus der Astronomie bekannten Konzept des Leitsterns auf, so dass die annähernd punktförmige, virtuelle Lichtquelle im Folgenden auch als "Leitstern" bezeichnet wird.

In Unterverfahrensschritt iii) erfolgt die Detektion der räumlichen Intensitätsverteilung des reflektierten Lichts, also des durch die Übertragung mittels des von der virtuellen Lichtquelle distal beleuchteten Bereichs des Faserbündels gestörten Lichts, oder die Detektion eines Interferenzmusters, das durch kohärente Überlagerung des reflektierten, also des durch die Übertragung mittels des distal beleuchteten Bereichs des Faserbündels gestörten, Lichts mit einer Referenzwelle erzeugt wird. Dabei erlauben die detektierten Daten erfindungsgemäß aufgrund der distalen Beleuchtung der Fasern durch den Leitstern die Extraktion der einfachen Phasenverschiebung ΔΦ statt der doppelten Phasenverschiebung 2·ΔΦ bei rein proximaler Beleuchtung.

Prinzipiell ist es möglich, eine einzelne Faser des Faserbündels proximal zu beleuchten und die Mittel zur teilweisen Reflexion so weit von den distalen Facetten des Faserbündels entfernt anzuordnen, dass der Leitstern in genügend weiter Entfernung zu den distalen Facetten des Faserbündels entsteht, um das gesamte Faserbündel zu beleuchten. Dann kann Unterverfahrensschritt vi) entfallen, da durch die Unterverfahrensschritte i) bis v) bereits eine die Übertragungseigenschaften des gesamten Bündels optischer Fasern widerspiegelnde Systemfunktion ermittelt wurde. Ein großer Abstand zwischen den Mitteln zur teilweisen Reflexion und den distalen Facetten des Faserbündels kann allerdings zu Abbildungsfehlern führen und die Qualität der Kalibrierung negativ beeinflussen. Vorteilhaft wird das erfindungsgemäße Verfahren daher so angewendet, dass der Abstand zwischen den Mitteln zur teilweisen Reflexion und den distalen Facetten des Faserbündels so gewählt wird, dass nur ein Teilbereich des Faserbündels um die proximal beleuchtete Einzelfaser distal beleuchtet wird. Die Unterverfahrensschritte i) bis v) werden so oft wiederholt, bis eine die Übertragungseigenschaften des gesamten Bündels optischer Fasern widerspiegelnde Systemfunktion ermittelt wurde. Dazu werden in Unterverfahrensschritt i) jeweils voneinander verschiedene Einzelfasern proximal beleuchtet, so dass verschiedene Teilbereiche des Faserbündels distal beleuchtet werden, wobei jeder Teilbereich zumindest einmal erfasst und die Systemfunktion des gesamten Bündels durch Kombination der Systemfunktionen der Teilbereiche berechnet wird. Der Abstand der Mittel zur teilweisen Reflexion von den distalen Facetten des Faserbündels kann typischerweise etwa 100 µm betragen.

Das Erzeugen mehrerer Leitsterne bietet den Vorteil einer genaueren Kalibrierung mit guter Fokusqualität. Um die Dauer der Kalibrierung vorteilhaft zu verkürzen, wird in einer Ausführungsform des erfindungsgemäßen Verfahrens der Unterverfahrensschritt i) so ausgeführt, dass mindestens zwei nicht benachbart angeordnete Einzelfasern des Bündels optischer Fasern gleichzeitig proximal beleuchtet werden, wobei in Unterverfahrensschritt ii) mindestens zwei virtuelle Bilder der Einzelfasem entstehen, mittels derer nichtüberlappende Bereiche des Faserbündels distal beleuchtet werden.

Zur Beobachtung des Objekts weist das erfindungsgemäße Verfahren folgenden Verfahrensschritt b) auf:
b) Störungskorrigierte proximale Beleuchtung des gesamten Bündels optischer Fasern mit Licht und Detektion des im optischen Weg nach den Mitteln zur teilweisen Reflexion angeordneten Objekts, oder
störungskorrigierte Detektion des im optischen Weg nach den Mitteln zur teilweisen Reflexion angeordneten Objekts nach unkorrigierter proximaler Beleuchtung des gesamten Bündels optischer Fasern mit Licht,
wobei die Störungskorrektur der Beleuchtung dadurch vorgenommen wird, dass dem von einem Beleuchtungssystem emittierten Licht durch Wechselwirkung mit einem einzeln ansteuerbare Elemente aufweisenden Wellenfrontmodulator das Inverse der Systemfunktion aufgeprägt wird, so dass nach Übertragung durch das Bündel optischer Fasern ein weitgehend dem von dem Beleuchtungssystem emittierten entsprechendes Wellenfeld zur proximalen Beleuchtung eines im optischen Weg nach dem Mittel zur teilweisen Reflexion angeordneten Objekts zur Verfügung steht;
wobei die Störungskorrektur der Detektion dadurch vorgenommen wird, dass die räumliche Intensitätsverteilung des durch die Übertragung mittels des Bündels optischer Fasern gestörten Lichts nach Wechselwirkung mit dem Objekt oder die räumliche Intensitätsverteilung eines durch kohärente Überlagerung des durch die Übertragung mittels des Bündels optischer Fasern gestörten Lichts nach Wechselwirkung mit dem Objekt mit einer Referenzwelle erzeugten Interferenzmusters durch Anwendung der inversen Systemfunktion numerisch korrigiert wird.

Nach der in Verfahrensschritt a) erfolgten Kalibrierung des Faserbündels, mittels derer die die Übertragungseigenschaften des gesamten Faserbündels widerspiegelnde Systemfunktion ermittelt wird, kann die Störungskorrektur vor der Beleuchtung des Objekts mittels des Faserbündels oder nach der Beleuchtung des Objekts mittels des Faserbündels vorgenommen werden.

Um die bei Übertragung des Lichts durch das Faserbündel hervorgerufene Störung zu kompensieren, wird das Objekt also entweder mit einer mittels eines Wellenfrontmodulators gemäß der inversen Systemfunktion vorverzerrten Wellenfront beleuchtet, oder die Beleuchtung erfolgt mit einer nichtvorverzerrten Wellenfront, deren Verzerrung durch das Faserbündel numerisch nach der Streuung am Objekt ausgeglichen wird. Als "Wellenfrontmodulator" ist im Sinne dieser Erfindung eine Einrichtung zur gezielten Beeinflussung der Phase und/oder der Amplitude einer Lichtwelle zu verstehen.

In einer Ausführungsform des erfindungsgemäßen Verfahrens wird in Unterverfahrensschritt i) die proximale Beleuchtung mindestens einer frei wählbaren Einzelfaser mittels mindestens einer ersten Beleuchtungsquelle durchgeführt, und in Verfahrensschritt b) die störungskorrigierte oder unkorrigierte proximale Beleuchtung des gesamten kohärenten Bündels optischer Fasern mittels einer zweiten Beleuchtungsquelle durchgeführt, oder es wird in Unterverfahrensschritt i) die proximale Beleuchtung mindestens einer frei wählbaren Einzelfaser und in Verfahrensschritt b) die störungskorrigierte oder unkorrigierte proximale Beleuchtung des gesamten Bündels optischer Fasern mittels genau einer Beleuchtungsquelle durchgeführt, wobei zumindest zur proximalen Beleuchtung mindestens einer frei wählbaren Einzelfaser das Licht mit einem im optischen Weg zwischen der Beleuchtungsquelle und dem kohärenten Bündel optischer Fasern angeordneten Wellenfrontmodulator wechselwirkt. Die einzeln ansteuerbaren Elemente des Wellenfrontmodulators werden dann so ausgerichtet, dass das Licht der Beleuchtungsquelle zu einem überwiegenden Anteil nicht das Faserbündel trifft, sondern nur der zur Beleuchtung mindestens einer Einzelfaser verwendete Anteil, wobei auch eine gleichzeitige Beleuchtung mehrerer nichtbenachbarter Einzelfasern gemäß der oben beschriebenen Ausführungsform auf besonders vorteilhafte Weise durchgeführt werden kann.

Die Häufigkeit, mit der die Kalibrierung des Faserbündels durchzuführen ist, wird im Allgemeinen durch die charakteristische Zeitskala der durch das Faserbündel hervorgerufenen Störung bestimmt, da die Systemfunktion bei Verfahrensschritt a) und Verfahrensschritt b) zumindest weitgehend gleich sein sollte. Die Durchführung von Verfahrensschritt a) und Verfahrensschritt b) kann dabei in beliebiger sequentieller Abfolge erfolgen, z. B. a) - b) - a) - b) oder a) - b) - b) - b) -a) - b) - b) - b). Ausführungsformen der Erfindung sind auch zur gleichzeitigen Durchführung von Verfahrensschritt a) und Verfahrensschritt b) geeignet, insbesondere, wenn die Störungskorrektur der Detektion numerisch vorgenommen wird.

Eine Ausführungsform des erfindungsgemäßen Verfahrens sieht vor, dass das Licht auf distaler Seite des Bündels optischer Fasern in Abhängigkeit von seiner Wellenlänge zu einem hohen Anteil reflektiert oder zu einem hohen Anteil transmittiert wird. Diese Ausgestaltung erlaubt vorteilhaft die gleichzeitige Durchführung der Kalibrierung des Faserbündels und der Beobachtung eines Objekts mittels des Faserbündels, indem zur Kalibrierung Licht einer Wellenlänge, welches zu einem hohen Anteil am distalen Ende des Faserbündels reflektiert wird, verwendet wird, und zur Beobachtung des Objekts Licht einer anderen Wellenlänge, welches zu einem hohen Anteil am distalen Ende des Faserbündels transmittiert wird. Durch dieses Vorgehen kann eine saubere Trennung detektierter Signale hinsichtlich ihres optischen Wegs anhand der Wellenlänge durchgeführt werden.

Eine weitere Ausführungsform des erfindungsgemäßen Verfahrens sieht vor, dass das Licht auf distaler Seite des Bündels optischer Fasern in Abhängigkeit von seinem Polarisationszustand zu einem hohen Anteil reflektiert oder zu einem hohen Anteil transmittiert wird. Auch diese Ausgestaltung erlaubt vorteilhaft die gleichzeitige Durchführung der Kalibrierung des Faserbündels und der Beobachtung eines Objekts mittels des Faserbündels, indem zur Kalibrierung Licht eines Polarisationszustands, welches zu einem hohen Anteil am distalen Ende des Faserbündels reflektiert wird, verwendet wird, und zur Beobachtung des Objekts Licht eines anderen Polarisationszustands, welches zu einem hohen Anteil am distalen Ende des Faserbündels transmittiert wird. Durch dieses Vorgehen kann eine saubere Trennung detektierter Signale hinsichtlich ihres optischen Wegs anhand des Polarisationszustands durchgeführt werden.

Eine weitere Ausführungsform des erfindungsgemäßen Verfahrens ist dadurch gekennzeichnet, dass die Auswertung der detektierten Daten und die Extraktion zumindest der Phaseninformation des reflektierten, durch die Übertragung mittels des distal beleuchteten Bereichs des Faserbündels gestörten Lichts durch digitale Holografie erfolgt. Detektiert und ausgewertet wird dabei die räumliche Intensitätsverteilung eines Interferenzmusters, das durch kohärente Überlagerung des reflektierten, gestörten Lichts mit einer Referenzwelle entsteht. Besonders bevorzugt wird die Referenzwelle durch Strahlteilung des vom Beleuchtungssystem emittierten Lichts erzeugt. Vorteilhaft bietet die digitale Holografie die Möglichkeit, schnell und ohne aufwändige Iterationen die Phaseninformation zu bestimmen. Die Frequenz der Störungskorrektur ist im Wesentlichen baugruppenlimitiert, beispielsweise durch die Frequenz, mit der die Einstellung der Elemente des Wellenfrontmodulators vorgenommen werden kann.

In einer weiteren Ausführungsform des erfindungsgemäßen Verfahrens wird die Auswertung der detektierten Daten und die Extraktion zumindest der Phaseninformation des reflektierten, durch die Übertragung mittels des distal beleuchteten Bereichs des Faserbündels gestörten Lichts aus der detektierten Intensitätsverteilung durch numerische Rekonstruktion mittels eines Phase-retrieval-Verfahrens vorgenommen. Es kann beispielsweise eine Auswertung der Transport-of-Intensity Equation erfolgen, wobei dazu die Detektion der Intensitätsverteilung an mindestens zwei verschiedenen Punkten entlang des optischen Wegs vorgenommen werden muss.

In einer weiteren Ausführungsform wird das erfindungsgemäße Verfahren dahingehend erweitert, dass ein über einen weiten Parameterbereich frei einstellbares Lichtmuster, insbesondere auch mit mehreren Brennpunkten, auf distaler Seite des Faserbündels erzeugt wird. Dazu erfolgt eine entsprechende Ansteuerung der einzeln ansteuerbaren Elemente eines proximal angeordneten Wellenfrontmodulators. Mit diesem kann auch gleichzeitig die Störungskompensation der Beleuchtung durchgeführt werden. Vorteilhaft können durch die Möglichkeit zur Fokussierung mittels des Wellenfrontmodulators Tiefeninformationen über ein Beobachtungsobjekt gewonnen werden. Es können Scans durchgeführt werden, und das Verfahren kann für aktorische Anwendungen von Licht genutzt werden, z. B. für optische Pinzetten oder therapeutische Bestrahlungen.

Neben dem beschriebenen Verfahren umfasst die Erfindung auch ein zugehöriges faseroptisches System.

Das erfindungsgemäße faseroptische System zur Beleuchtung und Detektion eines Objekts mit Licht weist ein Beleuchtungssystem auf, mit dem ein kohärentes Bündel optischer Fasern, beispielsweise Glasfasern, beleuchtet wird, wobei das Faserbündel insbesondere biegsam gestaltet ist.

Im optischen Weg zwischen den distalen Facetten, also den dem Objekt zugewandten und von dem Beleuchtungssystem abgewandten Stirnseiten der Fasern des Faserbündels, und dem zu beobachtenden Objekt sind Mittel zur teilweisen Reflexion des von dem Beleuchtungssystem emittierten Lichts angeordnet. Die Mittel zur teilweisen Reflexion sind von den distalen Facetten beabstandet, also nicht unmittelbar an den distalen Facetten anliegend (beispielsweise nicht aufgedampft).

Des Weiteren weist das erfindungsgemäße faseroptische System Mittel zur Detektion der räumlichen Intensitätsverteilung des durch die Übertragung mittels des Faserbündels gestörten Lichts oder zur Detektion eines Interferenzmusters, das durch kohärente Überlagerung des durch die Übertragung mittels des Bündels optischer Fasern gestörten Lichts mit einer Referenzwelle erzeugt wird, auf. Die Störung kann beispielsweise als Verzerrung der Wellenfronten des Lichts bzw. als Deformation des Wellenfelds veranschaulicht werden.

Außerdem weist das erfindungsgemäße faseroptische System eine Signalverarbeitungsplattform zumindest zur Auswertung der detektierten Daten und zur Ermittlung zumindest der Phaseninformation des durch die Übertragung mittels des Faserbündels gestörten Lichts auf.

Neben den genannten Komponenten kann das erfindungsgemäße System auch weitere, in zweckmäßiger Weise angeordnete optische Komponenten, insbesondere Strahlteiler, aufweisen.

Erfindungsgemäß ist das Beleuchtungssystem dazu ausgebildet, sowohl zur Kalibrierung des faseroptischen Systems mindestens eine frei wählbare Einzelfaser des Bündels optischer Fasern zu beleuchten, als auch zur Detektion des Objekts das gesamte Bündel optischer Fasern zu beleuchten. Die Kalibrierung des faseroptischen Systems umfasst dabei zumindest die Erfassung und Auswertung der Systemfunktion des Faserbündels, die insbesondere durch die Transmissionsmatrix zwischen dem proximalen und dem distalen Ende des Faserbündels dargestellt werden kann.

Mit dem erfindungsgemäßen faseroptischen System kann vorteilhaft sowohl dessen Kalibrierung, als auch die Detektion und Beobachtung des Objekts in-situ und instantan durchgeführt werden, so dass Änderungen der Systemfunktion, die beispielsweise durch Bewegung, Vibration oder Temperaturänderung hervorgerufen werden, ohne aufwändige manuelle Justage sogleich erfasst werden können.

Wie bereits bei der Beschreibung des erfindungsgemäßen Verfahrens ausgeführt, beruht die erfindungsgemäße Lösung im Wesentlichen darauf, zur Kalibrierung des faseroptischen Systems direkt die einfache Phasenverschiebung Δφ bei Durchgang des vom Beleuchtungssystem emittierten Lichts zu messen, und die so ermittelte Systemfunktion zur Störungskorrektur bei der Beobachtung des Objekts zu verwenden, indem entweder das Inverse der Systemfunktion auf die proximale Beleuchtung des gesamten Faserbündels angewendet oder das Inverse der Systemfunktion zur numerischen Korrektur der das Objekt charakterisierenden Messdaten verwendet wird.

Vorteilhaft kann das erfindungsgemäße faseroptische System zur Durchführung von Abbildungen eingesetzt werden, deren laterale Auflösung nicht mehr durch den Pixelabstand des Faserbündels (üblicherweise > 3,3 µm), sondern nur noch durch die numerische Apertur der lichtführenden Faserkerne limitiert ist. In Abhängigkeit vom Durchmesser der Faserkerne ist damit eine laterale Auflösung von weniger als 1 µm erzielbar.

Bevorzugt weist das erfindungsgemäße faseroptische System nur auf der proximalen Seite des Faserbündels eine Instrumentierung auf. Am distalen Ende des Faserbündels werden keine aktiven oder vergrößernden optischen Elemente benötigt. Dadurch ist es vorteilhaft möglich, mittels der Erfindung ultradünne Endoskope herzustellen, deren Durchmesser auf distaler Seite im Wesentlichen durch den Durchmesser des Faserbündels bestimmt wird.

In einer Ausführungsform des erfindungsgemäßen faseroptischen Systems umfasst das Beleuchtungssystem mindestens eine Beleuchtungsquelle und einen mehrere einzeln ansteuerbare Elemente aufweisenden Wellenfrontmodulator, wobei der Wellenfrontmodulator im optischen Weg zwischen den proximalen Facetten der optischen Fasern des Bündels und der mindestens einen Beleuchtungsquelle angeordnet ist. Die Elemente des Wellenfrontmodulators sind so ausrichtbar, dass zur Kalibrierung des faseroptischen Systems mindestens eine frei wählbare Einzelfaser des Bündels optischer Fasern mittels der mindestens einen Beleuchtungsquelle proximal beleuchtet wird. Der nicht zur Beleuchtung der mindestens einen Einzelfaser benötigte Anteil des Beleuchtungslichts wird mittels des Wellenfrontmodulators so abgelenkt, dass er das Faserbündel nicht trifft. "Mindestens eine Einzelfaser" bedeutet im Sinne der Erfindung, dass zum Zwecke der Kalibrierung auch mehrere nichtbenachbarte Einzelfasern des Faserbündels gleichzeitig oder nacheinander proximal beleuchtet werden können; es bedeutet nicht, dass bei der Kalibrierung ein mehrere Fasern umfassender Bereich um eine Einzelfaser herum gleichzeitig mit der Einzelfaser proximal beleuchtet wird. Vorteilhaft kann das Beleuchtungssystem durch Ansteuerung verschiedener Elemente des Wellenfrontmodulators auf besonders einfache Weise zur Kalibrierung durch gleichzeitige oder zeitlich aufeinanderfolgende Beleuchtung mehrerer Einzelfasern verwendet werden.

Das Beleuchtungssystem kann gemäß einer weiteren Ausführungsform des erfindungsgemäßen Systems auch mindestens zwei Beleuchtungsquellen umfassen, wobei mittels mindestens einer ersten, zur Kalibrierung des faseroptischen Systems verwendeten Beleuchtungsquelle mindestens eine frei wählbare Einzelfaser des Faserbündels beleuchtet wird, und mittels einer zweiten, zur Detektion des Objekts verwendeten Beleuchtungsquelle das gesamte Bündel optischer Fasern beleuchtet wird. Vorteilhaft kann bei dieser Ausführungsform die gesamte Intensität der zur Kalibrierung verwendeten Beleuchtungsquelle ausgenutzt werden.

Als Beleuchtungsquelle wird beispielsweise ein Laser verwendet.

In einer weiteren Ausführungsform des erfindungsgemäßen faseroptischen Systems ist ein mehrere einzeln ansteuerbare Elemente aufweisender Wellenfrontmodulator im optischen Weg zwischen den proximalen Facetten der optischen Fasern des Bündels und dem Beleuchtungssystem angeordnet, wobei die Elemente des Wellenfrontmodulators von der Signalverarbeitungsplattform dergestalt ansteuerbar sind, dass durch Reflexion an den Elementen des Wellenfrontmodulators dem von dem Beleuchtungssystem zur Beleuchtung des Objekts emittierten, ungestörten Licht das Inverse der durch die Übertragung mittels des Bündels optischer Fasern erzeugten Störung aufgeprägt wird.

In einer Ausgestaltung dieser Ausführungsform des erfindungsgemäßen faseroptischen Systems ist vorgesehen, dass mittels der einzeln ansteuerbaren Elemente des Wellenfrontmodulators ein über einen weiten Parameterbereich frei einstellbares Lichtmuster, also eine räumliche Intensitäts- und/oder Phasen- und/oder Polarisationszustandsverteilung, auf distaler Seite des Bündels optischer Fasern erzeugt wird. Insbesondere kann das Lichtmuster mehrere Brennpunkte aufweisen. Vorteilhaft wird dadurch ermöglicht, Scans mit dem faseroptischen System durchzuführen, Tiefeninformationen über ein Beobachtungsobjekt zu erhalten und das faseroptische System aktorisch einzusetzen, z. B. für optische Pinzetten oder therapeutische Bestrahlungen.

In einer weiteren Ausgestaltung dieser Ausführungsform des erfindungsgemäßen faseroptischen Systems ist vorgesehen, dass der Wellenfrontmodulator ein digitaler örtlicher Flächenlichtmodulator ist. Örtliche Flächenlichtmodulatoren umfassen im Allgemeinen eine Anordnung von LCoS- oder LCD-Zellen oder Mikrospiegeln auf einem Halbleiterchip, die einzeln angesteuert und gekippt und/oder abgesenkt werden können, sowie eine anwendungsspezifische integrierte Schaltung. Der Flächenlichtmodulator weist dabei zumindest ein Element, also einen Mikrospiegel, pro Faser des Faserbündels auf; üblich ist ein Verhältnis von etwa 100 Elementen pro Faser. Vorteilhaft kann die Verkippung oder Absenkung der einzelnen Mikrospiegel sehr schnell verändert werden, so dass instantan auf Änderungen der Systemfunktion reagiert werden kann.

Ausführungsformen des erfindungsgemäßen faseroptischen Systems sind dadurch gekennzeichnet, dass die Mittel zur teilweisen Reflexion des von dem Beleuchtungssystem emittierten Lichts eine Verspiegelung umfassen, deren Reflexionsgrad eine Funktion der Wellenlänge oder des Polarisationszustands ist, oder dass die Verspiegelung teildurchlässig ausgebildet ist.

In einer Ausführungsform des erfindungsgemäßen faseroptischen Systems umfassen die Mittel zur teilweisen Reflexion des vom Beleuchtungssystem emittierten Lichts eine Verspiegelung, deren Reflexionsgrad eine Funktion der Wellenlänge des Lichts ist, so dass die Verspiegelung als wellenlängenselektiver Strahlteiler wirkt. In Sinne der Erfindung dient die wellenlängenselektive Verspiegelung dazu, Licht einer bestimmten Wellenlänge bzw. eines Wellenlängenbereichs in hohem Maße zu reflektieren und Licht einer anderen bestimmten Wellenlänge oder eines anderen Wellenlängenbereichs in hohem Maße zu transmittieren. Eine solchermaßen wirkende, insbesondere mehrschichtig ausgeführte Verspiegelung wird auch als "dichroitischer Filter" bezeichnet. Der Vorteil dieser Ausgestaltung besteht insbesondere darin, dass bei Nutzung von Licht zweier verschiedener Wellenlängen, insbesondere mit geringem Wellenlängenunterschied, wobei eine Wellenlänge geringfügig kleiner und eine Wellenlänge geringfügig größer als die Kante des Filters gewählt wird, die Kalibrierung des faseroptischen Systems und die störungskorrigierte Beobachtung eines Objekts gleichzeitig durchgeführt werden können (ähnlich dem Wavelength Division Multiplexing). Es wird also gleichzeitig mit Licht mindestens einer Wellenlänge, die in hohem Maße transmittiert wird, das Objekt beleuchtet und vermessen, und mit Licht mindestens einer Wellenlänge, die in hohem Maße reflektiert wird, das faseroptische System kalibriert. Auch bei einer Speckle-Dekorrelation zwischen den verschiedenen Wellenlängen kann mit Hilfe einer bekannten Dispersionsrelation eine Korrektur vorgenommen werden.

In einer Ausführungsform des erfindungsgemäßen faseroptischen Systems umfassen die Mittel zur teilweisen Reflexion des vom Beleuchtungssystem emittierten Lichts eine Verspiegelung, deren Reflexionsvermögen eine Funktion des Polarisationszustands des Lichts ist, so dass die Verspiegelung als polarisationszustandsabhängiger Strahlteiler (auch als polarisierender Strahlteiler oder Polwürfel bezeichnet) wirkt, wobei ein Polarisationszustand des Lichts (z. B. rechtszirkular polarisiert) in hohem Maße reflektiert und ein anderer Polarisationszustand (z. B. linkszirkular polarisiert) in hohem Maße transmittiert wird. Es wird also gleichzeitig mit Licht eines Polarisationszustands, der in hohem Maße transmittiert wird, das Objekt beleuchtet und vermessen, und mit Licht eines Polarisationszustands, der in hohem Maße reflektiert wird, das faseroptische System kalibriert. Bei der beschriebenen bevorzugten Ausgestaltung können vorteilhaft die Kalibrierung des faseroptischen Systems und die störungskorrigierte Beobachtung eines Objekts gleichzeitig durchgeführt werden. Voraussetzung dafür ist, dass die Fasern des Faserbündels polarisationserhaltend ausgebildet sind; beispielsweise können Faserbündel mit photonischen Kristallfasern eingesetzt werden.

In einer Ausführungsform des erfindungsgemäßen faseroptischen Systems umfassen die Mittel zur teilweisen Reflexion des vom Beleuchtungssystem emittierten Lichts einen teildurchlässigen Spiegel, wobei die Eigenschaft "teildurchlässig" auch Konfigurationen des Spiegels umfasst, in denen das Licht nicht zu gleichen Anteilen reflektiert oder transmittiert wird. Bevorzugt kann das Licht zu einem überwiegenden Anteil von dem teildurchlässigen Spiegel transmittiert werden. Besonders bevorzugt wird nur ein Anteil der Lichtintensität im einstelligen Prozentbereich von dem teildurchlässigen Spiegel reflektiert und zur Kalibrierung des faseroptischen Systems verwendet, und der verbleibende Anteil wird transmittiert und zur Beobachtung des Objekts verwendet. In dieser Ausgestaltung kann in Abhängigkeit von der Laufzeit des Lichtsignals eine Trennung der zur Kalibrierung und der zur störungskorrigierten Beobachtung eines Objekts verwendeten Anteile vorgenommen werden.

Die Mittel zur teilweisen Reflexion des Lichts sind von den distalen Facetten des Faserbündels beabstandet angeordnet. Insbesondere sind die Mittel, beispielweise in den oben genannten Ausführungsformen, nicht direkt und unmittelbar auf die distalen Facetten des Faserbündels aufgedampft. Eine Beabstandung kann z. B. mittels eines Abstandstücks aus Glas erzielt werden, auf das distal eine Verspiegelung aufgebracht ist. Ein analoger Effekt wird dadurch erzielt, dass die Quarzglasfasern eines Faserbündels, deren Kern zur Brechzahl-Erhöhung mit Ge dotiert ist, distal einen undotierten Bereich aufweisen, auf den eine Verspiegelung aufgedampft wird. Die axiale Ausdehnung des undotierten Bereichs gibt dann den Abstand zwischen den distalen Facetten des Faserbündels und den Mitteln zur teilweisen Reflexion des Lichts vor.

In einer weiteren Ausführungsform des erfindungsgemäßen faseroptischen Systems umfassen die Mittel zur Detektion eine CCD-Kamera oder eine CMOS-Kamera. Die eingesetzten Kameras weisen dabei zumindest 1 Pixel pro Faser des Faserbündels auf; üblich ist ein Verhältnis von 10 bis 100 Pixeln pro Faser.

In einer weiteren Ausführungsform des erfindungsgemäßen faseroptischen Systems weist die Signalverarbeitungsplattform ein FPGA (field programmable gate array) oder einen Grafikprozessor (insbesondere unter Verwendung von GPGPU = General Purpose Computation on Graphics Processing Units) oder beides auf. Diese Komponenten des digitalen Regelungssystems zeichnen sich vorteilhaft durch geringe Latenzzeiten aus.

Erfindungsgemäß beschriebene Merkmale und Details des Systems gelten selbstverständlich auch im Zusammenhang mit dem erfindungsgemäßen Verfahren und umgekehrt.

Die Erfindung kann beispielsweise in faseroptischen Systemen Anwendung finden, die zur Abbildung eines Objekts vorgesehen sind, z. B. Endoskopen oder Mikroskopen. Die Erfindung kann aber auch Anwendung finden in faseroptischen Systemen, die z. B. für die lasergeführte Chirurgie, für Zellstimulation oder optogenetische Stimulation vorgesehen sind. Auch Anwendungen beim Additive Manufacturing sind möglich. Es versteht sich, dass diese Aufzählung nur einen Teil der vielfältigen Anwendungsmöglichkeiten der Erfindung wiedergeben kann.

Die Erfindung ist nicht auf die dargestellten und beschriebenen Ausführungsformen beschränkt, sondern umfasst auch alle im Sinne der Erfindung gleich wirkenden Ausführungsformen. Für die Realisierung der Erfindung ist es auch zweckmäßig, die vorbeschriebenen erfindungsgemäßen Ausgestaltungen, Ausführungsformen und einzelne Merkmale der Ansprüche miteinander zu kombinieren. Die Erfindung ist nicht auf die speziell beschriebenen Merkmalskombinationen beschränkt, sondern kann auch durch jede beliebige andere Kombination von bestimmten Merkmalen aller insgesamt offenbarten Einzelmerkmale definiert sein, sofern sich die Einzelmerkmale nicht gegenseitig ausschließen, oder eine spezifische Kombination von Einzelmerkmalen nicht explizit ausgeschlossen ist.

Im Folgenden wird die Erfindung anhand von Ausführungsbeispielen erläutert, ohne auf diese beschränkt zu sein.

Es zeigt die
Fig. 1 eine Prinzipskizze zur Ermittlung der Systemfunktion eines Faserbündels, wobei der Detektor auf der proximalen Seite des Faserbündels angeordnet ist, und die
Fig. 2 eine Prinzipskizze zur Kompensation der Systemfunktion des Faserbündels vor der Beleuchtung des Objekts.

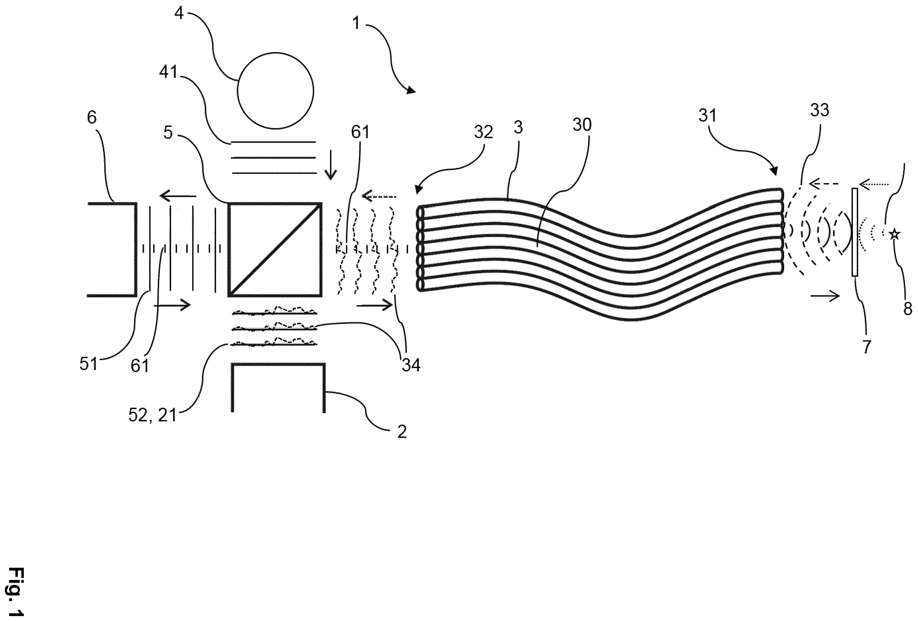

Fig. 1 zeigt eine Prinzipskizze eines faseroptischen Systems 1 mit einem Detektor 2 auf proximaler Seite 32 des biegsamen Faserbündels 3, mit der die Kalibrierung in Form der initialen Messung der Systemfunktion des Faserbündels 3 veranschaulicht werden soll. Die Ermittlung der Phaseninformation des durch die Übertragung mittels des Faserbündels 3 gestörten Lichts erfolgt im Ausführungsbeispiel über digitale Holografie.

Das Beleuchtungssystem umfasst eine Beleuchtungsquelle 4 und einen Flächenlichtmodulator 6. Von der Beleuchtungsquelle 4 ausgesandtes Licht, das durch die ebenen Wellenfronten 41 repräsentiert wird, wird in einem Strahlteiler 5 aufgeteilt in einen Anteil 51, der in Richtung des Flächenlichtmodulators 6 reflektiert wird, und einen zweiten Anteil 52. Der zweite Anteil 52 wird durch den Strahlteiler 5 direkt auf den Detektor 2 transmittiert und dient als Referenzwelle 21 für die Holografie.

Die Einzelelemente des Flächenlichtmodulators 6 sind so verkippt, dass von Anteil 51 des Beleuchtungslichts nur ein Anteil 61 in Form von ebenen Wellen in Richtung des Faserbündels 3 reflektiert wird, mittels dessen genau eine einzelne Faser 30 des Faserbündels 3 proximal beleuchtet wird. Auf distaler Seite 31 kann die Einzelfaser 30 als Ausgangspunkt einer Elementarwelle 33 aufgefasst werden, die die nahezu punktförmige Einzelfaser 30 verlässt und von einem Mittel zur teilweisen Reflexion in Form eines teildurchlässigen Spiegels 7 zumindest anteilig wieder in Richtung des Faserbündels 30 reflektiert wird. Dies entspricht optisch der Beleuchtung des Faserbündels von distaler Seite 31 aus mittels eines Leitsterns 8, der das hinter dem teildurchlässigen Spiegel 7 entstehende virtuelle Bild der nahezu punktförmigen Einzelfaser 30 darstellt. Letztlich wird also zumindest ein Bereich des Faserbündels 3 um die Einzelfaser 30 herum distal von der Elementarwelle 33 beleuchtet. Die Größe des Bereichs des Faserbündels 3, der auf diese Weise distal beleuchtet wird, ist abhängig vom Abstand zwischen der distalen Facette der Einzelfaser 30 und dem teildurchlässigen Spiegel 7. Fig. 1 zeigt eine Ausführung, in der das gesamte Faserbündel 3 distal beleuchtet wird.

Am proximalen Ende 32 verlassen durch die Übertragung mittels des Faserbündels 3 mit der einfachen Phasenverschiebung Δφ verzerrte Wellenfronten 34 das Faserbündel 3. Diese werden mittels des Strahlteilers 5 zumindest anteilig auf den Detektor 2, der z. B. eine CCD-Kamera sein kann, reflektiert und mit der Referenzwelle 21 überlagert, so dass der Detektor 2 ein Interferenzmuster aufzeichnet, über dessen digitale Auswertung in der Signalverarbeitungsplattform (nicht dargestellt) holografisch direkt die Phaseninformation der verzerrten Wellen 34 extrahiert werden kann. Damit kann die Systemfunktion, die die Übertragungseigenschaften des Faserbündels 3 repräsentiert, in Form einer Transfermatrix bestimmt werden.

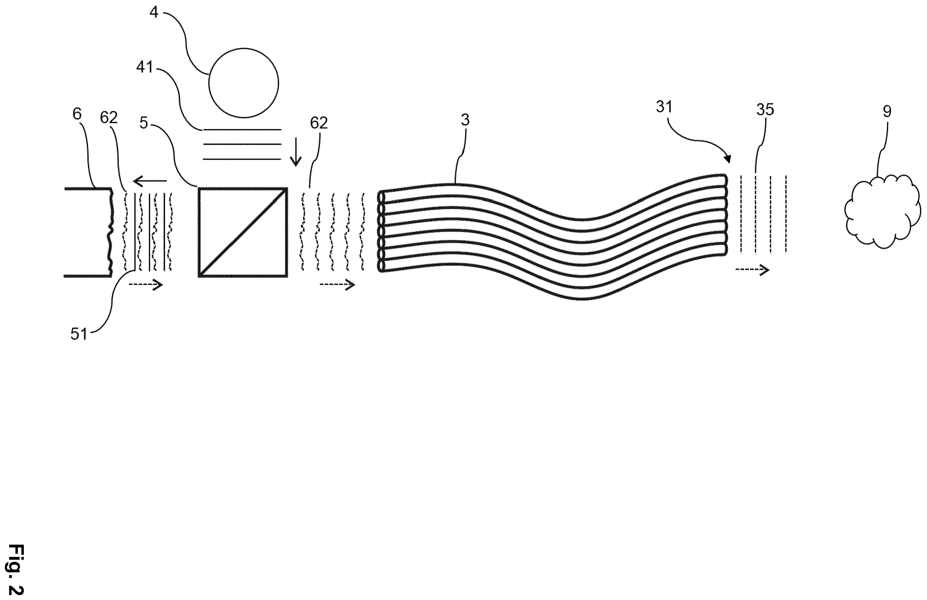

Fig. 2 zeigt eine Prinzipskizze zur Kompensation der Systemfunktion, die die Übertragungseigenschaften des Faserbündels 3 repräsentiert, mittels eines Flächenlichtmodulators 6 vor der Beleuchtung eines Objekts 9. Der Kompensation der Systemfunktion geht erfindungsgemäß jeweils deren Messung und Auswertung durch eine Signalverarbeitungsplattform (nicht dargestellt) voran, wobei ein Ausführungsbeispiel zur Kalibrierung des Faserbündels 3 in Fig. 1 beschrieben ist. Mittels der Signalverarbeitungsplattform wird die gemessene Systemfunktion invertiert und die Einstellung der einzelnen Elemente des Flächenlichtmodulators 6 dergestalt vorgenommen, dass dem von der Beleuchtungsquelle 4 emittierten Licht, hier wieder durch ebene Wellen 41 repräsentiert, welches vom Strahlteiler 5 zumindest anteilig auf den Flächenlichtmodulator 6 reflektiert wird, durch Reflexion am Flächenlichtmodulator 6 das Inverse der durch das Faserbündel 3 hervorgerufenen Phasenverzerrung aufgeprägt wird. Nach der Reflexion am Flächenlichtmodulator 6 weisen die Wellenfronten 62 eine Vorverzerrung auf, die bei der anschließenden Übertragung durch das Faserbündel 3 kompensiert wird. Am distalen Ende 31 treten ebene, störungskorrigierte Wellen 35 aus dem Faserbündel aus, die zur beugungslimitierten Beleuchtung eines Objekts 9 verwendet werden können. Die vor dem Objekt 9 angeordneten Mittel zur teilweisen Reflexion sind aus Gründen der Übersichtlichkeit nicht dargestellt.

Die Einzelelemente des Flächenlichtmodulators 6 können auch so positioniert sein, dass zum einen die Phasenverzerrung, die die ebenen Wellen 51 durch das Faserbündel 3 erfahren, kompensiert wird. Zum anderen erfolgt die Positionierung der Einzelelemente des Flächenlichtmodulators 6 dergestalt, dass bei distalem Austritt des Lichts aus dem Faserbündel 3 eine Fokussierung des Lichts in einem Brennpunkt oder mehreren Brennpunkten resultiert (nicht dargestellt). Bei Reflexion der ebenen Wellen 51 am Flächenlichtmodulator 6 wird diesen also die invertierte Systemfunktion aufgeprägt, und es erfolgt eine Fokussierung. Die so vordeformierten und fokussierten Wellenfronten (nicht dargestellt) werden dann in das Faserbündel 3 geleitet, wo die Phasenvorverzerrung unter Beibehaltung der Fokussierung kompensiert wird. Distal verlassen fokussierte, störungskorrigiert unverzerrte Wellen das Faserbündel 3.

### Bezugszeichen

- 1: Faseroptisches System
- 2: Detektor
- 21: Referenzwelle
- 3: Faserbündel
- 30: Einzelfaser
- 31: Distale Seite des Faserbündels
- 32: Proximale Seite des Faserbündels
- 33: Wellen zur distalen Beleuchtung des Faserbündels
- 34: Durch Faserbündel verzerrte Wellen, am proximalen Ende austretend
- 35: Störungskorrigierte Wellen
- 4: Beleuchtungsquelle
- 41: Von der Beleuchtungsquelle emittierte ebene Wellen
- 5: Strahlteiler
- 51: Von Strahlteiler in Richtung des Flächenlichtmodulators reflektierte Wellen
- 52: Durch Strahlteiler transmittierte Wellen
- 6: Flächenlichtmodulator
- 61: Von Flächenlichtmodulator reflektierte Wellen zur Beleuchtung einer Einzelfaser
- 62: Vom Flächenlichtmodulator reflektierte, vorverzerrte Wellen
- 7: Teildurchlässiger Spiegel
- 8: Leitstern
- 9: Objekt

## Patentansprüche

1. Verfahren zur Beleuchtung und Detektion eines Objekts (9) mit Licht, aufweisend zumindest folgende Verfahrensschritte:
a) Kalibrierung eines kohärenten Bündels optischer Fasern (3), umfassend
i) Proximale Beleuchtung mindestens einer frei wählbaren Einzelfaser (30) des kohärenten Bündels optischer Fasern (3) mit Licht;
ii) Zumindest teilweise Reflexion des Lichts an von den distalen Facetten der optischen Fasern des Bündels (3) beabstandet angeordneten Mitteln zur teilweisen Reflexion (7) des Lichts, so dass von dem reflektierten Licht ein mehr als eine Einzelfaser (30) umfassender Bereich des Bündels optischer Fasern (3) distal beleuchtet wird, wobei das reflektierte Licht dem von dem virtuellen Bild (8) der beleuchteten Einzelfaser (30) ausgehenden Licht entspricht;
iii) Detektion der räumlichen Intensitätsverteilung des reflektierten, durch die Übertragung mittels des distal beleuchteten Bereichs des Bündels optischer Fasern (3) gestörten Lichts oder der räumlichen Intensitätsverteilung eines durch kohärente Überlagerung des reflektierten, durch die Übertragung mittels des distal beleuchteten Bereichs des Bündels optischer Fasern (3) gestörten Lichts mit einer Referenzwelle erzeugten Interferenzmusters;
iv) Auswertung der detektierten Daten zumindest zur direkten Extraktion der einfachen Phasenverschiebung ΔΦ des reflektierten, durch die Übertragung mittels des distal beleuchteten Bereichs des Bündels optischer Fasern (3) gestörten Lichts;
v) Ermittlung einer die Übertragungseigenschaften des distal beleuchteten Bereichs des Bündels optischer Fasern (3) widerspiegelnden Systemfunktion;
vi) Wiederholung der Unterverfahrensschritte i) bis v) zur Ermittlung einer die Übertragungseigenschaften des gesamten Bündels optischer Fasern (3) widerspiegelnden Systemfunktion;
b) Störungskorrigierte proximale Beleuchtung des gesamten Bündels optischer Fasern (3) mit Licht und Detektion des im optischen Weg nach den Mitteln zur teilweisen Reflexion (7) angeordneten Objekts (9), oder
störungskorrigierte Detektion des im optischen Weg nach den Mitteln zur teilweisen Reflexion (7) angeordneten Objekts (9) nach unkorrigierter proximaler Beleuchtung des gesamten Bündels optischer Fasern (3) mit Licht,
wobei die Störungskorrektur der Beleuchtung dadurch vorgenommen wird, dass dem von einem Beleuchtungssystem emittierten Licht durch Wechselwirkung mit einem einzeln ansteuerbare Elemente aufweisenden Wellenfrontmodulator (6) das Inverse der Systemfunktion aufgeprägt wird, so dass nach Übertragung durch das Bündel optischer Fasern (3) ein weitgehend dem von dem Beleuchtungssystem emittierten entsprechendes Wellenfeld zur proximalen Beleuchtung eines im optischen Weg nach dem Mittel zur teilweisen Reflexion (7) angeordneten Objekts (9) zur Verfügung steht;
wobei die Störungskorrektur der Detektion dadurch vorgenommen wird, dass die räumliche Intensitätsverteilung des durch die Übertragung mittels des Bündels optischer Fasern (3) gestörten Lichts nach Wechselwirkung mit dem Objekt (9) oder die räumliche Intensitätsverteilung eines durch kohärente Überlagerung des durch die Übertragung mittels des Bündels optischer Fasern (3) gestörten Lichts nach Wechselwirkung mit dem Objekt (9) mit einer Referenzwelle erzeugten Interferenzmusters durch Anwendung der inversen Systemfunktion numerisch korrigiert wird.

2. Verfahren nach Anspruch 1, **dadurch gekennzeichnet, dass** in Unterverfahrensschritt i) mindestens zwei nicht benachbart angeordnete Einzelfasern (30) des Bündels optischer Fasern (3) gleichzeitig proximal beleuchtet werden, wobei in Unterverfahrensschritt ii) mindestens zwei virtuelle Bilder der Einzelfasern (30) entstehen, mittels derer nichtüberlappende Bereiche des Bündels optischer Fasern (3) distal beleuchtet werden.

3. Verfahren nach Anspruch 1 oder 2, **dadurch gekennzeichnet, dass** in Unterverfahrensschritt i) die proximale Beleuchtung mindestens einer frei wählbaren Einzelfaser (30) mittels mindestens einer ersten Beleuchtungsquelle durchgeführt wird, und in Verfahrensschritt b) die störungskorrigierte oder unkorrigierte proximale Beleuchtung des gesamten kohärenten Bündels optischer Fasern (3) mittels einer zweiten Beleuchtungsquelle durchgeführt wird, oder
dass in Unterverfahrensschritt i) die proximale Beleuchtung mindestens einer frei wählbaren Einzelfaser (30) und in Verfahrensschritt b) die störungskorrigierte oder unkorrigierte proximale Beleuchtung des gesamten Bündels optischer Fasern (30) mittels genau einer Beleuchtungsquelle (4) durchgeführt wird, wobei zumindest zur proximalen Beleuchtung mindestens einer frei wählbaren Einzelfaser (30) das Licht mit einem im optischen Weg zwischen der Beleuchtungsquelle (4) und dem kohärenten Bündel optischer Fasern (3) angeordneten Wellenfrontmodulator (6) wechselwirkt.

4. Verfahren nach einem der Ansprüche 1 bis 3, **dadurch gekennzeichnet, dass** das Licht auf distaler Seite des Bündels optischer Fasern (3) durch die Mittel zur teilweisen Reflexion (7) in Abhängigkeit von seiner Wellenlänge zu einem hohen Anteil reflektiert oder zu einem hohen Anteil transmittiert wird.

5. Verfahren nach einem der Ansprüche 1 bis 3, **dadurch gekennzeichnet, dass** das Licht auf distaler Seite des Bündels optischer Fasern (3) durch die Mittel zur teilweisen Reflexion (7) in Abhängigkeit von seinem Polarisationszustand zu einem hohen Anteil reflektiert oder zu einem hohen Anteil transmittiert wird.

6. Verfahren nach einem der Ansprüche 1 bis 5, **dadurch gekennzeichnet, dass** die Auswertung der detektierten Daten und die Extraktion zumindest der Phaseninformation des reflektierten, durch die Übertragung mittels des Bündels optischer Fasern (3) gestörten Lichts aus der räumlichen Intensitätsverteilung eines durch kohärente Überlagerung des reflektierten, durch die Übertragung mittels des Bündels optischer Fasern (3) gestörten Lichts mit einer Referenzwelle erzeugten Interferenzmusters durch digitale Holografie erfolgt.

7. Verfahren nach einem der Ansprüche 1 bis 5, **dadurch gekennzeichnet, dass** die Auswertung der detektierten Daten und die Extraktion zumindest der Phaseninformation des reflektierten, durch die Übertragung mittels des Bündels optischer Fasern (3) gestörten Lichts aus der räumlichen Intensitätsverteilung durch numerische Rekonstruktion mittels eines Phase-retrieval-Verfahrens erfolgt.

8. Verfahren nach einem der Ansprüche 1 bis 7, **dadurch gekennzeichnet, dass** ein frei einstellbares Lichtmuster auf distaler Seite des Bündels optischer Fasern erzeugt (3) wird.

9. Faseroptisches System (1) zur Beleuchtung und Detektion eines Objekts (9) mit Licht, aufweisend
- ein kohärentes Bündel optischer Fasern (3);
- ein Beleuchtungssystem zur proximalen Beleuchtung des kohärenten Bündels optischer Fasern (3);
- Mittel zur teilweisen Reflexion (7) des Lichts, die im optischen Weg des von dem Beleuchtungssystem emittierten Lichts zwischen den distalen Facetten der optischen Fasern des Bündels (3) und dem Objekt (9) von den distalen Facetten beabstandet angeordnet sind;
- Mittel zur Detektion (2) der räumlichen Intensitätsverteilung des durch die Übertragung mittels des Bündels optischer Fasern (3) gestörten Lichts oder der räumlichen Intensitätsverteilung eines durch kohärente Überlagerung des durch die Übertragung mittels des Bündels optischer Fasern (3) gestörten Lichts mit einer Referenzwelle erzeugten Interferenzmusters;
- eine Signalverarbeitungsplattform zumindest zur Auswertung der detektierten Daten und zur Ermittlung zumindest der Phaseninformation des durch die Übertragung mittels des Bündels optischer Fasern (3) gestörten Lichts;
wobei das Beleuchtungssystem dazu ausgebildet ist, zur Kalibrierung des Bündels optischer Fasern (3) mindestens eine frei wählbare Einzelfaser (30) des Bündels optischer Fasern (3) zu beleuchten, und zur Beobachtung des Objekts (9) das gesamte Bündel optischer Fasern (3) zu beleuchten.

10. Faseroptisches System (1) nach Anspruch 9, **dadurch gekennzeichnet, dass** das Beleuchtungssystem mindestens eine Beleuchtungsquelle (4) und einen mehrere einzeln ansteuerbare Elemente aufweisenden Wellenfrontmodulator (6) umfasst, wobei der Wellenfrontmodulator (6) im optischen Weg zwischen den proximalen Facetten der optischen Fasern des Bündels (3) und der mindestens einen Beleuchtungsquelle (4) angeordnet ist, wobei die Elemente des Wellenfrontmodulators (6) so ausrichtbar sind, dass zur Kalibrierung des Bündels optischer Fasern (3) mindestens eine frei wählbare Einzelfaser (30) des Bündels optischer Fasern (3) mittels der mindestens einen Beleuchtungsquelle (4) beleuchtet wird.

11. Faseroptisches System nach Anspruch 9, **dadurch gekennzeichnet, dass** das Beleuchtungssystem mindestens zwei Beleuchtungsquellen umfasst, wobei mittels mindestens einer ersten Beleuchtungsquelle zur Kalibrierung des Bündels optischer Fasern (3) mindestens eine frei wählbare Einzelfaser (30) des Bündels optischer Fasern (3) beleuchtet wird, und mittels einer zweiten Beleuchtungsquelle zur Beobachtung des Objekts (9) das gesamte Bündel optischer Fasern (3) beleuchtet wird.

12. Faseroptisches System (1) nach einem der Ansprüche 9 bis 11, **dadurch gekennzeichnet, dass** ein mehrere einzeln ansteuerbare Elemente aufweisender Wellenfrontmodulator (6) im optischen Weg zwischen den proximalen Facetten der optischen Fasern des Bündels (3) und dem Beleuchtungssystem angeordnet ist, wobei die Elemente des Wellenfrontmodulators (6) von der Signalverarbeitungsplattform dergestalt ansteuerbar sind, dass durch Reflexion an den Elementen des Wellenfrontmodulators (6) dem von dem Beleuchtungssystem zur Beleuchtung und Detektion des Objekts (9) emittierten, ungestörten Licht das Inverse der durch die Übertragung mittels des Bündels optischer Fasern (3) erzeugten Störung aufgeprägt wird.

13. Faseroptisches System (1) nach Anspruch 12, **dadurch gekennzeichnet, dass** mittels der einzeln ansteuerbaren Elemente des Wellenfrontmodulators (6) auf distaler Seite des Bündels optischer Fasern (3) ein einstellbares Lichtmuster erzeugt wird.

14. Faseroptisches System (1) nach Anspruch 12 oder 13, **dadurch gekennzeichnet, dass** der Wellenfrontmodulator (6) ein digitaler örtlicher Flächenlichtmodulator ist.

15. Faseroptisches System (1) nach einem der Ansprüche 9 bis 14, **dadurch gekennzeichnet, dass** die Mittel zur teilweisen Reflexion (7) des Lichts eine Verspiegelung umfassen, die als wellenlängenabhängiger Strahlteiler wirkt.

16. Faseroptisches System (1) nach einem der Ansprüche 9 bis 14, **dadurch gekennzeichnet, dass** die Mittel zur teilweisen Reflexion (7) des Lichts eine Verspiegelung umfassen, die als polarisationszustandsabhängiger Strahlteiler wirkt.

17. Faseroptisches System (1) nach einem der Ansprüche 9 bis 14, **dadurch gekennzeichnet, dass** die Mittel zur teilweisen Reflexion (7) des Lichts eine teildurchlässige Verspiegelung umfassen.

18. Faseroptisches System (1) nach einem der Ansprüche 9 bis 17, **dadurch gekennzeichnet, dass** die Mittel zur Detektion (2) eine CCD-Kamera oder eine CMOS-Kamera umfassen.

19. Faseroptisches System (1) nach einem der Ansprüche 9 bis 18, **dadurch gekennzeichnet, dass** die Signalverarbeitungsplattform ein FPGA (field programmable gate array) oder einen Grafikprozessor oder beides aufweist.

## Claims

1. Method for illuminating and detecting an object (9) by light, comprising at least the following method steps:
a) calibrating a coherent bundle of optical fibres (3), comprising
i) proximal illumination of at least one freely selectable individual fibre (30) of the coherent bundle of optical fibres (3) with light;
ii) reflecting the light at least in part at means for partial reflection (7) of the light that are spaced apart from the distal facets of the optical fibres of the bundle (3), such that a region of the bundle of optical fibres (3) that comprises more than one individual fibre (30) is illuminated distally by the reflected light, wherein the reflected light corresponds to the light emanating from the virtual image (8) of the illuminated individual fibres (30);
iii) detecting the spatial intensity distribution of the reflected light subjected to disturbance by being transmitted by means of the distally illuminated region of the bundle of optical fibres (3), or the spatial intensity distribution of an interference pattern generated by coherent superposing a reference wave on the reflected light subjected to disturbance by being transmitted by means of the distally illuminated region of the bundle of optical fibres (3);
iv) evaluating the detected data at least in order to directly extract the single phase difference ΔΦ of the reflected light subjected to disturbance by being transmitted by means of the distally illuminated region of the bundle of optical fibres (3);
v) determining a system function that mirrors the transmission properties of the distally illuminated region of the bundle of optical fibres (3);
vi) repeating sub-method steps i) to v) in order to determine a system function that mirrors the transmission properties of the entire bundle of optical fibres (3);
b) disturbance-corrected proximal illumination of the entire bundle of optical fibres (3) by means of light, and detection of the object (9) arranged in the optical path after the means for partial reflection (7), or
disturbance-corrected detection of the object (9) arranged in the optical path after the means for partial reflection (7) following uncorrected proximal illumination of the entire bundle of optical fibres (3) by means of light,
wherein the disturbance correction of the illumination is carried out in that the inverse of the system function is impressed on the light emitted by an illumination system, by means of interaction with a wavefront modulator (6) comprising individually actuatable elements, such that, following transfer through the bundle of optical fibres (3), a wave field that largely corresponds to that emitted by the illumination system is available for proximal illumination of an object (9) arranged in the optical path after the means for partial reflection (7);
wherein the disturbance correction of the detection is carried out in that the spatial intensity distribution of the light subjected to disturbance by being transmitted by means of the bundle of optical fibres (3), following interaction with the object (9), or the spatial intensity distribution of an interference pattern generated by coherently superposing a reference wave on the light subjected to disturbance by being transmitted by means of the bundle of optical fibres (3), following interaction with the object (9), is numerically corrected by means of applying the inverse system function;

2. Method according to claim 1, **characterised in that**, in sub-method step i), at least two non-adjacent individual fibres (30) of the bundle of optical fibres (3) are proximally illuminated at the same time, at least two virtual images of the individual fibres (30) resulting in sub-method step ii), by means of which virtual images non-overlapping regions of the bundle of optical fibres (3) are illuminated distally.

3. Method according to either claim 1 or claim 2, **characterised in that**, in sub-method step i), at least one freely selectable individual fibre (30) is illuminated proximally by means of at least one first illumination source, and in method step b) the disturbance-corrected or uncorrected proximal illumination of the entire coherent bundle of optical fibres (3) is carried out by means of a second illumination source or,
in sub-method step i) the proximal illumination of at least one freely selectable individual fibre (30) and in method step b) the disturbance-corrected or uncorrected proximal illumination of the entire bundle of optical fibres (30) are carried out using exactly one illumination source (4), the light interacting with a wavefront modulator (6) that is arranged in the optical path between the illumination source (4) and the coherent bundle of optical fibres (3), at least for the purpose of proximal illumination of at least one freely selectable individual fibre (30).

4. Method according to any of claims 1 to 3, **characterised in that** the means for partial reflection (7) largely reflect or largely transmit the light on the distal side of the bundle of optical fibres (3), depending on the wavelength thereof.

5. Method according to any of claims 1 to 3, **characterised in that** the means for partial reflection (7) largely reflect or largely transmit the light on the distal side of the bundle of optical fibres (3), depending on the polarisation state thereof.

6. Method according to any of claims 1 to 5, **characterised in that** the evaluation of the detected data and the extraction at least of the phase information of the reflected light subjected to disturbance by being transmitted by means of the bundle of optical fibres (3) is carried out by means of digital holography from the spatial intensity distribution of an interference pattern generated by coherently superposing a reference wave on the reflected light that is subjected to disturbance by being transmitted by means of the bundle of optical fibres (3).

7. Method according to any of claims 1 to 5, **characterised in that** the evaluation of the detected data and the extraction, from the spatial intensity distribution, at least of the phase information of the reflected light subjected to disturbance by being transmitted by means of the bundle of optical fibres (3) is carried out by means of numerical reconstruction, by means of a phase retrieval method.

8. Method according to any of claims 1 to 7, **characterised in that** a freely adjustable light pattern is generated on the distal side of the bundle of optical fibres (3).

9. Fibre-optical system (1) for illuminating and detecting an object (9) by means of light, comprising
- a coherent bundle of optical fibres (3);
- an illumination system for proximally illuminating the coherent bundle of optical fibres (3);
- means for partial reflection (7) of the light, which means are arranged in the optical path of the light emitted by the illumination system, between the distal facets of the optical fibres of the bundle (3) and the object (9) at a spacing from the distal facets;
- means for detecting (2) the spatial intensity distribution of the light subjected to disturbance by being transmitted by means of the bundle of optical fibres (3), or the spatial intensity distribution of an interference pattern generated by coherently superposing a reference wave on the light subjected to disturbance by being transmitted by means of the bundle of optical fibres (3);
- a signal processing platform at least for evaluating the detected data and for determining at least the phase information of the light that is subjected to disturbance by being transmitted by means of the bundle of optical fibres (3);
wherein the illumination system is designed to illuminate at least one freely selectable individual fibre (30) of the bundle of optical fibres (3) in order to calibrate the bundle of optical fibres (3), and to illuminate the entire bundle of optical fibres (3) in order to observe the object (9).

10. Fibre-optical system (1) according to claim 9, **characterised in that** the illumination system comprises at least one illumination source (4) and a wavefront modulator (6) that comprises a plurality of individually actuatable elements, the wavefront modulator (6) being arranged in the optical path between the proximal facets of the optical fibres of the bundle (3) and the at least one illumination source (4), the elements of the wavefront modulator (6) being able to be oriented such that, in order to calibrate the bundle of optical fibres (3), at least one freely selectable individual fibre (30) of the bundle of optical fibres (3) is illuminated by means of the at least one illumination source (4).

11. Fibre-optical system according to claim 9, **characterised in that** the illumination system comprises at least two illumination sources, at least one freely selectable individual fibre (30) of the bundle of optical fibres (3) being illuminated by means of at least one first illumination source in order to calibrate the bundle of optical fibres (3), and the entire bundle of optical fibres (3) being illuminated by means of a second illumination source in order to observe the object (9).

12. Fibre-optical system (1) according to any of claims 9 to 11, **characterised in that** a wavefront modulator (6) comprising a plurality of individually actuatable elements is arranged in the optical path between the proximal facets of the optical fibres of the bundle (3) and the illumination system, the elements of the wavefront modulator (6) being able to be actuated by the signal processing platform in such a way that the inverse of the disturbance generated by the transmission by means of the bundle of optical fibres (3) is impressed on the undisturbed light emitted by the illumination system for illuminating and detecting the object (9), by means of reflection at the elements of the wavefront modulator (6).

13. Fibre-optical system (1) according to claim 12, **characterised in that** an adjustable light pattern is generated on the distal side of the bundle of optical fibres (3) by means of the individually actuatable elements of the wavefront modulator (6).

14. Fibre-optical system (1) according to either claim 12 or claim 13, **characterised in that** the wavefront modulator (6) is a digital local spatial light modulator.

15. Fibre-optical system (1) according to any of claims 9 to 14, **characterised in that** the means for partial reflection (7) of the light comprise a reflective surface that functions as a wavelength-dependent beam splitter.

16. Fibre-optical system (1) according to any of claims 9 to 14, **characterised in that** the means for partial reflection (7) of the light comprise a reflective surface that functions as a polarisation state-dependent beam splitter.

17. Fibre-optical system (1) according to any of claims 9 to 14, **characterised in that** the means for partial reflection (7) of the light comprise a semitransparent reflective surface.

18. Fibre-optical system (1) according to any of claims 9 to 17, **characterised in that** the means for detection (2) comprise a CCD camera or a CMOS camera.

19. Fibre-optical system (1) according to any of claims 9 to 18, **characterised in that** the signal processing platform comprises an FPGA (field programmable gate array) or a graphics processing unit or both.

## Revendications

1. Procédé d'éclairage et de détection d'un objet (9) par la lumière, comportant au moins les étapes suivantes :
a) étalonnage d'un faisceau cohérent de fibres optiques (3), comprenant
i) l'éclairage proximal, par la lumière, d'au moins une fibre individuelle (30) librement sélectionnable du faisceau cohérent de fibres optiques (3) ;
ii) la réflexion au moins partielle de la lumière sur des moyens de réflexion partielle (7) de la lumière disposés à distance des facettes distales des fibres optiques du faisceau (3), de sorte qu'une section du faisceau de fibres optiques (3) comprenant plus d'une fibre individuelle (30) est éclairée de manière distale par la lumière réfléchie, la lumière réfléchie correspondant à la lumière émanant de l'image virtuelle (8) de la fibre individuelle (30) éclairée ;
iii) la détection de la répartition spatiale de l'intensité de la lumière réfléchie perturbée par la transmission au moyen de la zone éclairée de manière distale du faisceau de fibres optiques (3) ou de la répartition spatiale de l'intensité d'un motif d'interférence produit par une onde de référence grâce à la superposition cohérente de la lumière réfléchie perturbée par la transmission au moyen de la zone éclairée de manière distale du faisceau de fibres optiques (3) ;
iv) l'évaluation des données détectées au moins pour l'extraction directe du déphasage simple (ΔΦ) de la lumière réfléchie perturbée par la transmission au moyen de la zone éclairée de manière distale du faisceau de fibres optiques (3) ;
v) la détermination d'une fonction du système reflétant les caractéristiques de transmission de la zone éclairée de manière distale du faisceau de fibres optiques (3) ;
vi) la répétition des sous-étapes i) à v) pour déterminer une fonction du système reflétant les caractéristiques de transmission de tout le faisceau de fibres optiques (3) ;
b) éclairage proximal à perturbations corrigées de tout le faisceau de fibres optiques (3) par la lumière, la détection de l'objet (9) disposé dans le chemin optique après les moyens de réflexion partielle (7), ou la détection à perturbations corrigées de l'objet (9) disposé dans le chemin optique après les moyens de réflexion partielle (7) après un éclairage proximal non corrigé de tout le faisceau de fibres optiques (3) par la lumière,
la correction des perturbations de l'éclairage étant effectuée en appliquant l'inverse de la fonction du système à la lumière émise par un système d'éclairage par interaction avec un modulateur de front d'onde (6) comportant des éléments pouvant être commandés individuellement, de sorte que, après la transmission au moyen du faisceau de fibres optiques (3), un champ d'onde correspondant dans une large mesure à celui émis par le système d'éclairage est disponible pour l'éclairage proximal d'un objet (9) disposé dans le chemin optique après le moyen de réflexion partielle (7) ;
la correction des perturbations de la détection étant effectuée en corrigeant numériquement la répartition spatiale de l'intensité de la lumière perturbée par la transmission au moyen du faisceau de fibres optiques (3) après l'interaction avec l'objet (9) ou de la répartition spatiale de l'intensité d'un motif d'interférence produit par une onde de référence grâce à la superposition cohérente de la lumière perturbée par la transmission au moyen du faisceau de fibres optiques (3) après l'interaction avec l'objet (9) avec une onde de référence par l'application de la fonction de système inverse.

2. Procédé selon la revendication 1, **caractérisé en ce que**, à la sous-étape i), au moins deux fibres individuelles non adjacentes (30) du faisceau de fibres optiques (3) sont simultanément éclairées de manière proximale, dans lequel, à la sous-étape ii), au moins deux images virtuelles des fibres individuelles (30) sont générées, au moyen desquelles des zones non chevauchantes du faisceau de fibres optiques (3) sont éclairées de manière distale.

3. Procédé selon la revendication 1 ou 2, **caractérisé en ce que**, à la sous-étape i), l'éclairage proximal d'au moins une fibre individuelle (30) librement sélectionnable est effectué au moyen d'au moins une première source d'éclairage, et à l'étape b), l'éclairage proximal à perturbations corrigées ou non de tout le faisceau cohérent de fibres optiques (3) est effectué au moyen d'une seconde source d'éclairage, ou **en ce que**, à la sous-étape i), l'éclairage proximal d'au moins une fibre individuelle (30) librement sélectionnable et, à l'étape b), l'éclairage proximal à perturbations corrigées ou non de tout le faisceau de fibres optiques (30) est effectué au moyen d'exactement une source d'éclairage (4), la lumière interagissant, au moins pour l'éclairage proximal d'au moins une fibre individuelle (30) librement sélectionnable, avec un modulateur de front d'onde (6) disposé dans le chemin optique entre la source d'éclairage (4) et le faisceau cohérent de fibres optiques (3).

4. Procédé selon l'une des revendications 1 à 3, **caractérisé en ce que** la lumière sur le côté distal du faisceau de fibres optiques (3) est réfléchie ou transmise dans une proportion élevée par le moyen de réflexion partielle (7) en fonction de sa longueur d'onde.

5. Procédé selon l'une des revendications 1 à 3, **caractérisé en ce que** la lumière sur le côté distal du faisceau de fibres optiques (3) est réfléchie ou transmise dans une proportion élevée par le moyen de réflexion partielle (7) en fonction de son état de polarisation.

6. Procédé selon l'une des revendications 1 à 5, **caractérisé en ce que** l'évaluation des données détectées et l'extraction d'au moins l'information de phase de la lumière réfléchie perturbée par la transmission au moyen du faisceau de fibres optiques (3) à partir de la répartition spatiale de l'intensité d'un motif d'interférence produit par une onde de référence grâce à la superposition cohérente de la lumière réfléchie perturbée par la transmission au moyen du faisceau de fibres optiques (3) sont effectuées par holographie numérique.

7. Procédé selon l'une des revendications 1 à 5, **caractérisé en ce que** l'évaluation des données détectées et l'extraction d'au moins l'information de phase de la lumière réfléchie perturbée par la transmission au moyen du faisceau de fibres optiques (3) à partir de la répartition spatiale de l'intensité sont effectuées grâce à une reconstruction numérique au moyen d'un procédé d'extraction par phase.

8. Procédé selon l'une des revendications 1 à 7, **caractérisé en ce qu'**un motif lumineux librement réglable est généré (3) sur le côté distal du faisceau de fibres optiques.

9. Système à fibre optique (1) destiné à éclairer et à détecter un objet (9) par la lumière, comportant
- un faisceau cohérent de fibres optiques (3) ;
- un système d'éclairage pour l'éclairage proximal du faisceau cohérent de fibres optiques (3) ;
- des moyens de réflexion partielle (7) de la lumière, disposés dans le chemin optique de la lumière émise par le système d'éclairage entre les facettes distales des fibres optiques du faisceau (3) et l'objet (9) à une distance des facettes distales ;
- des moyens de détection (2) de la répartition spatiale de l'intensité de la lumière perturbée par la transmission au moyen du faisceau de fibres optiques (3) ou de la répartition spatiale de l'intensité d'un motif d'interférence produit par une onde de référence grâce à la superposition cohérente de la lumière perturbée par la transmission, au moyen du faisceau de fibres optiques (3) ;
- une plate-forme de traitement de signal au moins destinée à évaluer les données détectées et à déterminer au moins l'information de phase de la lumière perturbée par la transmission au moyen du faisceau de fibres optiques (3) ;
le système d'éclairage étant conçu pour éclairer au moins une fibre individuelle (30) librement sélectionnable du faisceau de fibres optiques (3) pour l'étalonnage du faisceau de fibres optiques (3), et pour éclairer tout le faisceau de fibres optiques (3) pour l'observation de l'objet (9).

10. Système à fibre optique (1) selon la revendication 9, **caractérisé en ce que** le système d'éclairage comprend au moins une source d'éclairage (4) et un modulateur de front d'onde (6) comportant une pluralité d'éléments pouvant être commandés individuellement, le modulateur de front d'onde (6) étant disposé dans le chemin optique entre les facettes proximales des fibres optiques du faisceau (3) et l'au moins une source d'éclairage (4), les éléments du modulateur de front d'onde (6) pouvant être orientés de telle manière que, pour l'étalonnage du faisceau de fibres optiques (3), au moins une fibre individuelle (30) librement sélectionnable du faisceau de fibres optiques (3) est éclairée au moyen de l'au moins une source d'éclairage (4).

11. Système à fibre optique selon la revendication 9, **caractérisé en ce que** le système d'éclairage comprend au moins deux sources d'éclairage, au moins une fibre individuelle (30) librement sélectionnable du faisceau de fibres optiques (3) étant éclairée au moyen d'au moins une première source d'éclairage pour l'étalonnage du faisceau de fibres optiques (3), et tout le faisceau de fibres optiques (3) étant éclairé au moyen d'une seconde source d'éclairage pour l'observation de l'objet (9).

12. Système à fibre optique (1) selon l'une des revendications 9 à 11, **caractérisé en ce qu'**un modulateur de front d'onde (6) comportant une pluralité d'éléments pouvant être commandés individuellement est disposé dans le chemin optique entre les facettes proximales des fibres optiques du faisceau (3) et le système d'éclairage, les éléments du modulateur de front d'onde (6) pouvant être commandés par la plate-forme de traitement de signal de telle manière que, par réflexion sur les éléments du modulateur de front d'onde (6), l'inverse de la perturbation générée par la transmission au moyen du faisceau de fibres optiques (3) est appliqué à la lumière non perturbée émise par le système d'éclairage pour l'éclairage et la détection de l'objet (9).

13. Système à fibre optique (1) selon la revendication 12, **caractérisé en ce qu'**un motif lumineux réglable est généré sur le côté distal du faisceau de fibres optiques (3) au moyen des éléments individuellement réglables du modulateur de front d'onde (6).

14. Système à fibre optique (1) selon la revendication 12 ou 13, **caractérisé en ce que** le modulateur de front d'onde (6) est un modulateur de lumière en surface numérique local.

15. Système à fibre optique (1) selon l'une des revendications 9 à 14, **caractérisé en ce que** les moyens de réflexion partielle (7) de la lumière comprennent une couche réfléchissante qui sert de séparateur de faisceau dépendant de la longueur d'onde.

16. Système à fibre optique (1) selon l'une des revendications 9 à 14, **caractérisé en ce que** les moyens de réflexion partielle (7) de la lumière comprennent une couche réfléchissante qui sert de séparateur de faisceau dépendant de l'état de polarisation.

17. Système à fibre optique (1) selon l'une des revendications 9 à 14, **caractérisé en ce que** les moyens de réflexion partielle (7) de la lumière comprennent une couche réfléchissante semi-transparente.

18. Système à fibre optique (1) selon l'une des revendications 9 à 17, **caractérisé en ce que** les moyens de détection (2) comprennent une caméra CCD ou une caméra CMOS.

19. Système à fibre optique (1) selon l'une des revendications 9 à 18, **caractérisé en ce que** la plate-forme de traitement de signal comporte un réseau prédiffusé programmable par l'utilisateur (FPGA) ou un processeur graphique ou les deux.
